# EUROPEAN PATENT SPECIFICATION

(11) **EP 3 662 902 B1**
(45) Date of publication and mention of the grant of the patent: **31.07.2024**
(21) Application number: 18211154.2
(22) Date of filing: 07.12.2018
(51) Int. Cl.: A61K 9/28, A61K 31/136, A61K 31/606, A61P 1/00, A61P 29/00

(54) **COLONIC DRUG DELIVERY FORMULATION**
FORMULIERUNG MIT FREISETZUNG EINES WIRKSTOFFS IM KOLON
FORMULATION POUR LA LIBERATION DE MÉDICAMENTS DANS LE CÔLON

(43) Date of publication of application: 10.06.2020
(73) Proprietor: Tillotts Pharma AG, 4310 Rheinfelden (CH)
(72) Inventor: VARUM, Felipe, 4310 Rheinfelden (CH); BRAVO GONZÁLEZ, Roberto Carlos, 4310 Rheinfelden (CH)
(74) Representative: Beck Greener LLP

(56) References cited:
- EP-A2- 0 673 645
- WO-A1-2013/164316
- WO-A1-98/16206
- US-A1- 2006 210 631
- US-A1- 2007 243 253

## Description

The present invention relates to a delayed release drug formulation with a core comprising a drug and a delayed release coating. In particular, it relates to a delayed release drug formulation for delivering a drug to the colon.

The targeting of drugs to the intestine is well known and has been known for over one hundred years. Commonly, the target of the drugs is the small intestine although the colon can be utilised as a means of achieving local therapy or systemic treatment. The requirements for the coatings on the drugs are different depending on the target site. In order to reach the colon, it is necessary for the drugs to pass through the small intestine, and therefore it is a requirement that a delayed release coating intended to release the drug in the colon does not release the drug in the small intestine.

Coated products for release in the small intestine commonly use polymer coatings which dissolve or disintegrate in a pH dependent manner. In the low pH environment of the stomach, the polymer coating is insoluble. However, on reaching the small intestine, the pH rises to 5 and above and the polymeric coating dissolves or disintegrates. A commonly used coating is one containing ionisable carboxylic groups. At higher pH levels, the carboxylic groups ionise, allowing the polymer coatings to disintegrate or dissolve. Common polymers of this type which are used include Eudragit^{®} L and Eudragit^{®} S.

Various methods of improving the release in the small intestine by ensuring an earlier release of the drug are known. US2008/0200482 is one of a number of references which discloses partially neutralizing the carboxylic groups in order to reduce the pH at which disintegration occurs. WO2008/135090 discloses a tablet with an inner coat of partially neutralized material and an outer coat with less or no neutralization. This is said to result in disintegration at an earlier time point when transferred from the stomach.

Release of drugs in the colon typically requires an alternative approach. The colon is susceptible to a number of disease states, including inflammatory bowel disease, irritable bowel syndrome, constipation, diarrhoea, infection and carcinoma. In such conditions, drug targeting to the colon would maximise the therapeutic effectiveness of the treatment. The colon can also be utilised as a portal for the entry of drugs into the systemic circulation. Various formulations have been developed for colonic drug delivery, including pro-drugs as well as formulated dosage forms, with the latter being more popular since the concept once proved can be applied to other drugs.

The higher bacterial population in the colon has also been exploited in developing colonic drug delivery dosage forms through the use, as digestable carrier materials, of naturally occurring polysaccharides that constitute substrates for the numerous enzymes produced by the resident colonic bacteria. These materials are typically able to pass through the upper gastrointestinal regions intact but are digested upon entry into the colon. Examples include starch, amylose, amylopectin, pectin, chitosan, galactomannan and guar gum.

One major attraction of using polysaccharides in this bacterial enzyme approach to colonic drug delivery is that materials used are of food grade and so would be safe for use in humans. They are usually applied as coatings or incorporated in the core material as a matrix carrier, and their digestion on entry into the colon by the colonic bacterial enzymes leads to the release of the drug load. An example of such a formulation, which employs an amylose coating, is disclosed in EP0343993A (BTG International Limited).

A major limitation with these naturally occurring materials, however, is that they swell excessively in aqueous media leading to leaching of the drug load in the upper gastrointestinal regions. To circumvent this problem, the naturally occurring materials have been utilised in a mixture with various impermeable materials.

EP0502032A (British Technology Group Ltd) teaches the use of an outer coating comprising a film-forming cellulose or acrylate polymer material and amorphous amylose for a tablet comprising an active compound. The polymer material used is a pH independent release polymer material.

An article in Journal of Controlled Release (Milojevic et al; 38; (1996); 75-84) reports the results of investigations concerning the incorporation of a range of insoluble polymers into an amylose coating in order to control amylose swelling. A range of cellulose and acrylate based co-polymers are assessed, and a commercially available ethyl cellulose (Ethocel^{®}) is found to control the swelling most effectively. A pH dependent soluble coating of Eudragit^{®} L 100 is employed but only in a multi-layer system comprising a bioactive coated with an inner coating of amylose and then an outer coating of Eudragit^{®} L 100.

A further amylose-based coating composition is disclosed in WO99/21536A (BTG International Limited). The coating composition comprises a mixture of amylose and a water-insoluble pH independent film-forming polymer which is formed from a water-insoluble cellulosic or acrylate polymer material.

WO99/25325A (BTG International Limited) also discloses a delayed release coating comprising amylose and (preferably) ethyl cellulose or alternatively an insoluble acrylate polymer. The coating composition also includes a plasticiser and the method finds particular application in the preparation of dosage forms comprising active materials that are unstable at temperatures in excess of 60 °C, as the composition is formed at lower temperatures than this.

WO03/068196A (Alizyme Therapeutics Ltd) discloses a specific delayed release coating for the bioactive prednisolone sodium metasulphobenzoate comprising glassy amylose, ethyl cellulose and dibutyl sebacate.

The use of polysaccharides other than amorphous amylose in a delayed release coating is disclosed in GB2367002 (British Sugar PLC). Examples include guar gum, karaya gum, gum tragacanth and xanthan gum. Microparticles of these polysaccharides are dispersed in a water-insoluble film-forming polymer matrix formed for example from a cellulose derivative, an acrylic polymer or a lignin.

WO01/76562A (Tampereen Patenttitoimisto Oy) discloses a peroral pharmaceutical formulation containing a drug and a chitosan (a polysaccharide obtained from chitin) for controlling its release. The drug and the chitosan are mixed into a homogeneous mechanical powder mixture which is granulated and then optionally tabletised. The granulation may be performed with an enteric polymer (such as a copolymer of methacrylic acid) or the granules may be provided with a porous enteric coating.

WO2004/052339A (Salvona LLC) discloses a pH dependent drug release system which is a free-flowing powder of solid hydrophobic nano-spheres comprising a drug encapsulated in a pH-sensitive micro-sphere. The nano-spheres are formed from the drug in combination with a wax material, and the pH-sensitive micro-sphere formed from a pH-sensitive polymer (such as a Eudragit^{®} polymer) in combination with a water-sensitive material such as a polysaccharide.

An article in the European Journal of Pharmaceutical Sciences (Akhgari et al; 28; March 2006; 307-314) reports the results of investigations into the use of certain polymethacrylate polymers to, *inter alia,* control the swelling of inulin. The polymethacrylate polymers tested were Eudragit^{®} RS; Eudragit^{®} RL; 1:1 mixtures of Eudragit^{®} RS and Eudragit^{®} RL; Eudragit^{®} FS; and 1:1 mixtures of Eudragit^{®} RS and Eudragit^{®} S.

US5422121 (Röhm GmbH) discloses an oral dosage form having a core containing at least one active ingredient enclosed within a shell material which comprises a polysaccharide that decomposes in the colon in admixture with a film-forming polymer. The ratio by weight of polysaccharide to film forming polymer is from 1:2 to 5:1, preferably from 1:1 to 4:1. Premature diffusion of the active ingredient from the core can be suppressed using a gastric resistant isolating layer. The reference exemplifies inter *alia* tablets having an inner isolating layer of Eudragit^{®} L30D with an outer layer comprising Eudragit^{®} L30D and guar gum (Example 2).

WO96/36321A discloses an oral dosage form comprising a core containing bisacodyl, and an enteric polymer coating for the core, the coating comprising at least one inner coating layer and an outer coating layer. The or each of the inner coating layer(s) is an enteric polymer that begins to dissolve in an aqueous medium at a pH from about 5 to about 6.3, and the outer coating layer is an enteric polymer that begins to dissolve in an aqueous medium at a pH from about 6.8 to about 7.2. The enteric polymer coating materials for the inner layer(s) are selected from the group consisting of cellulose acetate phthalate; cellulose acetate trimellitate; hydroxypropyl methylcellulose phthalate; hydroxypropyl methylcellulose acetate succinate; polyvinyl acetate phthalate; poly(methacrylic acid, methyl methacrylate) 1:1; poly(methacrylic acid, ethyl acrylate) 1:1; and compatible mixtures thereof.

WO2007/122374A discloses a colonic drug delivery formulation in which a mixture of a pH dependent film-forming polymeric material and a polysaccharide such as starch is used. Although it is known that this formulation shows delayed release followed by a quick release of the drug, it would be preferred if the drug release was quicker in the colon.

Both WO2013/164315A and WO2013/164316A disclose colonic drug delivery formulations in which a mixture of a pH dependent film forming polymeric material and a polysaccharide such as starch is used as an outer layer. An inner layer is used which is soluble in intestinal fluid or gastrointestinal fluid, being selected from a polycarboxylic acid polymer that is partially neutralised and a non-ionic polymer. The inner layer comprising the non-ionic polymer additionally comprises a buffer agent and/or a base.

In accordance with a first aspect of the present invention, there is provided a delayed release drug formulation for oral administration to deliver a drug to the colon of a subject, said formulation comprising:
a core, a coating for the core and an isolation layer between the core and the coating, the core comprising a drug and the coating comprising an outer layer and an inner layer,
wherein the outer layer comprises a film-forming enteric polymer having a pH threshold at pH 6 or above, and
wherein the inner layer comprises a film-forming non-ionic polymer that is soluble in intestinal or gastrointestinal fluid, and a buffer agent in an amount from more than 20 wt % to 60 wt %, based on the dry weight of the non-ionic polymer.

It has surprisingly been discovered that the delayed release drug formulations of the present invention advantageously show an unchanged release profile in Krebs buffer (pH 7.4) not only when the formulation has been pre-exposed to Fasted State Simulated Gastric Fluids (FaSSGF) but also when the formulation has been exposed to Fed State Simulated Gastric Fluids (FSSGF). This is advantageous as the release of the drug using the formulations of the present invention is relatively constant irrespective of the timing of taking the drug formulation or the diet of the user. Furthermore, the drug acceleration release mechanism is independent of the state of the stomach, *i.e.* whether the stomach is in the "fed" state or the "fasted" state.

It is a further advantage that the formulations of the present invention do not contain an intermediate layer containing an enteric material. Enteric materials are relatively expensive and therefore the absence of an intermediate layer containing such a material, whilst maintaining performance, will reduce the cost of producing the delayed release formulation. In addition, enteric polymers when neutralized to allow formation of aqueous coatings composition require a high quantity of plasticizer to allow a good film formation and to avoid brittleness.

A further technical advantage of the present invention (compared, for example, to the formulation disclosed in WO01/76562A) is that substantially no drug is released for an extended period (that is, whilst the coating is being dissolved), following which the drug is released relatively quickly. This is in contrast to sustained release matrix tablets described in WO01/76562A from which the drug release profile is gradual from the outset rather than delayed then pulsatile.

### Outer Layer

The outer layer comprises a film-forming enteric polymer having a pH threshold at about pH 6 or above. In preferred embodiments, the outer layer comprises a mixture of the enteric polymer and an enzymatically degradable polymer that is degraded by colonic enzymes.

It has been found that a mixture of two suitable polymers at an appropriate ratio, applied as a film coating on to a core, at least minimises, and can substantially eliminate, drug release in the stomach and small intestine. Subsequent drug release in the colon is believed to occur by the combined active physiological triggers: *i.e.* by dissolution of the second material, particularly Eudragit^{®} S, and digestion of the first material, e.g. starch or amylose.

The proportion of the enzymatically degradable polymer to the film-forming enteric polymer is typically at least 1:99, e.g. at least 10:90 and preferably at least 25:75. The proportion is typically no more than 99:1, e.g. no more than 75:25 and preferably no more than 60:40. In some embodiments, the proportion may be no more than 35:65. In some preferred embodiments, the proportion is from 10:90 to 75:25, e.g. from 10:90 to 60:40 and preferably from 25:75 to 60:40. In some particularly preferred embodiments, the proportion is from 15:85 to 35:65, e.g. from 25:75 to 35:65 and preferably about 30:70. In other particularly preferred embodiments, the proportion is from 40:60 to about 60:40, e.g. about 50:50.

The thickness of the outer layer coating of the core is typically from about 10 µm to about 300 µm. The thickness of a specific coating will, however, depend on the composition of the coating and on the size of the core. For example, coating thickness is directly proportional to the amount of polysaccharide in the coating.

Thus, in embodiments where the outer layer coating comprises high amylose starch and Eudragit^{®} S at a ratio of about 30:70, the coating thickness may be from about 70 µm to about 300 µm, and preferably from about 150 µm to about 250 µm. The thickness (in µm) for a given coating composition is dependent on core size.

The amount of enteric polymer in the outer layer is not related to the size of the core. The outer coating typically has a coating amount of enteric polymer of from about 2 mg/cm² to about 10 mg/cm², e.g. from about 2 mg/cm² to about 8 mg/cm², or from about 3 mg/cm² to about 8 mg/cm², or from about 4 mg/cm² to about 8 mg/cm², or from about 5 mg/cm² to about 8 mg/cm², or from about 6 mg/cm² to about 8 mg/cm², or from about 7 mg/cm² to about 8 mg/cm², e.g. about 7.5 mg/cm². This applies when the outer coating comprises only the film-forming enteric polymer and when the outer layer comprises a mixture of the film-forming enteric polymer and an enzymatically degradable polymer. A typical core has a diameter of from about 5 × 10⁻⁴ m to about 25 mm.

By saying that the outer coating comprises a mixture of the polysaccharide and enteric polymer, it is intended to exclude the known multi-layer dosage form (disclosed for example in Milojevic *et al.* described above) in which an active core is coated first with an inner coating of amylose and then with an outer coating of Eudragit^{®} L 100. In the context of the present invention, such a multi-layer dosage form does not comprise a mixture of starch and Eudragit^{®} L 100.

The outer coating is preferably a single layer of a mixture of the polysaccharide and enteric polymer, preferably a homogenous mixture.

The outer layer may optionally comprise one or more conventional excipients for polymer films, such as plasticisers for film formation (e.g. triethyl citrate (TEC)), surfactants (e.g. polysorbate 80), anti-tack agents (e.g. glyceryl monostearate), and pigments (e.g. iron oxide red or iron oxide yellow). These optional excipients may be included in amounts up to 30% by weight of the final composition of the polymer coating preparation.

### Film-forming enteric polymer

The outer layer comprises a film-forming enteric polymer having a pH threshold at about pH 6 or above. The film-forming enteric polymer is pH sensitive and has a pH threshold at about pH 6 or above. The "pH threshold" is the pH below which it is insoluble and at or above which it is soluble. The pH of the surrounding medium therefore triggers dissolution of the polymeric material. Thus, none (or essentially none) of the enteric polymer dissolves below the pH threshold. Once the pH of the surrounding medium reaches (or exceeds) the pH threshold, the second material becomes soluble.

By "insoluble" we mean that 1 g of the second material requires more than 10,000 ml of solvent (surrounding medium) to dissolve at a given pH.

By "soluble", we mean that 1 g of the second material requires less than 10,000 ml, preferably less than 5,000 ml, more preferably less than 1000 ml, even more preferably less than 100 ml or 10 ml of solvent to dissolve at a given pH.

"Surrounding medium" preferably means the medium in the gastrointestinal tract, such as the gastric juice or intestinal juice. Alternatively, the surrounding medium may be the *in vitro* equivalent of the medium in the gastrointestinal tract.

The normal pH of gastric juice is usually in the range of 1 to 3. The enteric polymer is insoluble below pH 6 and soluble at about pH 6 or above and, thus, is usually insoluble in gastric juice.

The pH of intestinal juice gradually increases from about 6 in the duodenum to about 7 to 8 in the distal small intestine. The enteric polymer is preferably insoluble below pH 6.5 (and soluble at about pH 6.5 or above) and, more preferably, is insoluble below pH 7 (and soluble at about pH 7 or above).

The pH threshold at which a material becomes soluble may be determined by a simple titration technique which would be part of the common general knowledge to the person skilled in the art.

Examples of suitable film-forming enteric polymers include an acrylate polymer, a cellulose polymer or a polyvinyl-based polymer. Examples of suitable cellulose polymers include cellulose acetate phthalate (CAP) and hydropropylmethylcellulose acetate succinate.

The film-forming enteric polymer is preferably a co-polymer of a (meth)acrylic acid and a (meth)acrylic acid C₁₋₄ alkyl ester, for instance, a copolymer of methacrylic acid and methacrylic acid methyl ester. Such a polymer is known as a poly(methacrylic acid/methyl methacrylate) co-polymer. Suitable examples of such co-polymers are usually anionic and not sustained release polymethacrylates. The ratio of carboxylic acid groups to methyl ester groups (the "acid:ester ratio") in these co-polymers determines the pH at which the co-polymer is soluble. The acid:ester ratio may be from about 2:1 to about 1:3, e.g. about 1:1 or, preferably, about 1:2. The molecular weight (MW) of preferred anionic co-polymers is usually from about 120,000 to 150,000, preferably about 135,000.

Preferred anionic poly(methacrylic acid/methyl methacrylate) co-polymers include Eudragit^{®} L (acid:ester ratio about 1:1; MW about 135,000; pH threshold of about 6); Eudragit^{®} S 100 (acid:ester ratio about 1:2; MW about 135,000; pH threshold of about 7); and Eudragit^{®} FS (a poly(methyl acrylate/methyl methacrylate/methacrylic acid); acid:ester ratio of about 1:10; MW about 220,000; pH threshold of about 7).

The film-forming enteric polymer may be a copolymer of methacrylic acid and ethyl acrylate. Eudragit^{®} L 100-55 poly(methacrylic acid/ethyl acrylate); acid:ester ratio of about 1:1; MW about 250,000; pH threshold of about 6. The Eudragit^{®} co-polymers are manufactured and/or distributed by Evonik, Darmstadt, Germany.

Mixtures of film-forming enteric polymers may be used as appropriate. An example of a suitable mixture would include a mixture, e.g. a 1:1 mixture, of Eudragit^{®} L and Eudragit^{®} S 100. However, the use of a particular film forming polymer material, e.g. a poly(methacrylic acid/methyl methacrylate) co-polymer, alone is preferred.

The use of Eudragit^{®} S 100 alone as the film-forming enteric polymer is particularly preferred.

Preferably, the exemplary polymers are used as the film-forming enteric polymer in at least partially neutralized form, *i.e.* that at least a portion, *e.g.* at least 10%, preferably between 15% and 20% (on a molar basis), more preferably at least 50%, and most preferably at least 90%, of the carboxylic acid groups in are in the form of carboxylate anions.

### Enzymatically degradable polymer

As discussed above, the outer layer may comprise a mixture of film-forming enteric polymer and an enzymatically degradable polymer that is degraded by colonic enzymes. The enzymatically degradable polymer is degraded by one or more bacterial enzymes found in the colon of a subject (colonic bacterial enzymes). Such enzymes are produced by colonic bacteria and include amylases such as alpha-amylases, beta-amylases and iso-amlayses; amylopullunase, glucoamylase, alpha-glucosidase, maltogenic-amylase, glycosyltransferases and amylomaltase.

The person skilled in the art is capable of determining whether a material is susceptible to attack by colonic bacterial enzymes using techniques comprising part of the common general knowledge. For example, a pre-determined amount of a given material could be exposed to an assay containing an enzyme from a bacterium found in the colon and the change in weight of the material over time may be measured. Alternatively, the amount of a degradation product produced as a result of the action of the colonic bacterial enzymes could be measured.

The enzymatically degradable polymer is preferably a polysaccharide. Suitable polysaccharides are selected from the group consisting of starch; amylose; amylopectin; chitosan; chondroitin sulfate; cyclodextrin; dextran; pullulan; carrageenan; sclerglucan; chitin; curdulan and levan. The polysaccharide is preferably starch. Starches are usually extracted from natural sources such as cereals; pulses; and tubers. Suitable starches for use in the present invention are typically food grade starches and include rice starch; wheat starch; corn (or maize) starch; pea starch; potato starch; sweet potato starch; tapioca starch; sorghum starch; sago starch; and arrow root starch. The use of maize starch is exemplified below.

Starch is typically a mixture of two different polysaccharides, namely amylose and amylopectin. Different starches may have different proportions of these two polysaccharides. Most natural (unmodified) maize starches have from about 20 wt % to about 30 wt % amylose with the remainder being at least substantially made up of amylopectin.

Suitable starches include "high amylose" and "low amylose" starches. High amylose starches are particularly preferred.

"High amylose" starches, are starches having at least 50 wt % amylose. Particularly suitable starches have from about 50 wt % to about 75 wt % amylose, preferably from about 50 wt % to about 70 wt %, more preferably from about 50 wt % to about 65 wt %, most preferably from about 50 wt % to about 60 wt %, e.g. about 55 wt %.

"Low amylose" starches are starches having less than 50 wt % amylose and at least 50 wt % amylopectin, e.g. up to 75 wt % amylopectin and even as much as up to 99 wt % amylopectin.

Starches suitable for use in the present invention typically have at least 0.1 wt %, e.g. at least 10 wt % or 15 wt %, preferably at least 35 wt %, amylose. Such starches have no more than 99.9 wt %, e.g. no more than 90 wt % or 85 wt %, preferably no more than 65 wt %, amylopectin. Such starches may have up to about 99 wt % amylose and no less than 1 wt % amylopectin.

Starches suitable for use in the present invention may have up to 100% amylopectin, more typically from about 0.1 wt % to about 99.9 wt % amylopectin. The starch may be, for instance, unmodified waxy corn starch. This typically comprises about 100% amylopectin.

Preferred starches have no more than 50 wt % amylopectin. Particularly suitable starches have from about 25 wt % to about 35 wt % amylopectin, e.g. about 30 wt % amylopectin.

The person skilled in the art is capable of determining the relative proportions of amylose and amylopectin in any given starch. For example, near-infrared (NIR) spectroscopy could be used to determine the amylose and amylopectin content of a starch using calibration curves obtained by NIR using laboratory-produced mixtures of known amounts of these two components. Further, starch could be hydrolysed to glucose using amyloglucosidase. A series of phosphorylation and oxidation reactions catalysed by enzymes result in the formation of reduced nicotinamide adenine dinucleotide phosphate (NADPH). The quantity of NADPH formed is stoichiometric with the original glucose content. Suitable test kits for this procedure are available (e.g., R-Biopharm GmbH, Germany). Another method that could be used involves subjecting the coating to digestion by bacterial enzymes, e.g. α-amylase, to produce short chain fatty acids (SCFA) which can be quantified by gas-liquid chromatography using a capillary column.

Preferred starches are "off-the-shelf" starches, *i.e.* starches which require no processing prior to use in the context of the present invention. Examples of particularly suitable "high amylose" starches include Eurylon^{®}6 (or IV) and Amylo N-400 (Roquette, Lestrem, France) or Amylogel 03003 (Cargill, Minneapolis, USA) all of which are examples of a maize starch having about 50 - 70 wt % amylose.

### Inner Layer

The inner layer comprises a film-forming non-ionic polymer which is soluble in gastrointestinal fluid (both gastric fluid and intestinal fluid), and a buffer agent in an amount of from more than about 20 wt % to about 60 wt % based on the dry weight of the non-ionic polymer.

As with the outer layer, the amount of polymer in the inner layer is not related to the size of the core. The inner layer typically has a coating amount of non-ionic polymer of from about 2 mg/cm² to about 5 mg/cm², e.g. about 3 mg/cm², based on the dry weight of the non-ionic polymer.

The inner layer may optionally comprise one or more conventional excipients for polymer films, such as plasticisers for film formation (e.g. triethyl citrate), surfactants (e.g. polysorbate 80) and anti-tack agents (e.g. glyceryl monostearate).

### Film-forming non-ionic polymer

The film-forming non-ionic polymer of the inner layer is soluble in intestinal fluid or gastrointestinal fluid (both gastric and intestinal fluid).

By "gastric fluid", the inventors mean the aqueous fluid in the stomach of a mammal, particularly a human. The fluid contains up to about 0.1 N hydrochloric acid and substantial quantities of potassium chloride and sodium chloride, and plays a key role in digestion by activating digestive enzymes and denaturing ingested protein. Gastric acid is produced by cells lining the stomach and other cells produce bicarbonate which acts as a buffer to prevent the gastric fluid from becoming too acidic.

By "intestinal fluid", the Inventors mean the fluid in the lumen of the intestine of a mammal, particularly a human. Intestinal fluid is a pale yellow aqueous fluid secreted from glands lining the walls of the intestine. Intestinal fluid includes fluid found in the small intestine, *i.e.* fluid found in the duodenum (or "duodenal fluid"), fluid found in the jejunum (or "jejunal fluid") and fluid found in the ileum (or "ileal fluid"), and fluid found in the large intestine, e.g. "colonic fluid".

The skilled person can readily determine whether a polymer is soluble in gastric fluid and/or intestinal fluid. If a polymer is soluble in water (or aqueous solution), e.g. a buffer solution) at a pH from 1 to 3, then that polymer would typically be soluble in gastric fluid. Similarly if a polymer is soluble in water (or aqueous solution, e.g. a buffer solution) at a pH from 5 to 8, then that polymer would typically be soluble in intestinal fluid. Alternatively, the compositions of gastric fluid and intestinal fluid are known and may be replicated *in vitro.* If a polymer is soluble in artificial gastric fluid or intestinal fluid *in vitro,* then it would typically be soluble in gastric fluid or intestinal fluid respectively *in vivo.*

The film-forming non-ionic polymer may be soluble in at least one fluid selected from gastric fluid, duodenal fluid, jejunal fluid and ileal fluid. However, in preferred embodiments, the solubility of the film-forming non-ionic polymer in water is not dependent on pH; at least not within the range of pH found in the intestine. In preferred embodiments, the film-forming non-ionic polymer is soluble in fluid at any point in the stomach and intestine, *i.e.* in gastrointestinal fluid.

It is preferred that the non-ionic polymer of the inner layer is a non-ionic cellulose-based polymer. Examples of suitable non-ionic cellulose-based polymers include methylcellulose (MC), hydroxypropyl cellulose (HPC), hydroxypropyl methylcellulose (HPMC). A particularly preferred non-ionic cellulose-based polymer is HPMC. Non-cellulose based polymers such as poly(ethyleneoxide)-graft-polyvinylalcohol, polyvinylpyrrolidinone (PVP), polyethylene glycol (PEG), PVP-grafted PEG and polyvinylalcohol (PVA) are also preferred. Mixtures of non-ionic polymers can be used. A particularly preferred mixture is HPMC and PEG.

### Base

In preferred embodiments, the inner layer comprises at least one base. The purpose of the base is to provide an alkaline environment on the underside of the outer layer once intestinal fluid begins to penetrate the outer layer. Without being bound by any particular theory, the Inventors believe that the alkaline environment facilitates dissolution and thereby also disintegration of the outer layer since the pH of the alkaline environment is above the pH threshold of the second polymeric material, thereby accelerating release of the drug from the formulation once the outer coating is dissolved and/or disintegrates.

In principle, any pharmacologically acceptable base may be used. The base is typically a non-polymeric compound. Suitable bases include inorganic bases such as sodium hydroxide, potassium hydroxide and ammonium hydroxide, and organic bases such as triethanolamine, sodium bicarbonate, potassium carbonate, trisodium phosphate, trisodium citrate or physiologically tolerated amines such as triethylamine.

The base is preferably selected from the group consisting of hydroxide bases, alkali metal bicarbonates, alkali metal carbonates, alkali metal phosphates, alkali metal citrates, or physiologically tolerated amines. More preferably, the base is a hydroxide base, and particularly preferred is sodium hydroxide.

The amount of base present in the inner layer would depend at least in part on the final pH of the inner coating preparation prior to coating a given batch of cores; the number of cores to be coated in the batch; the amount of the inner layer coating preparation used in the coating process of the batch.

### Buffer agent

The inner layer comprises at least one buffer agent. The purpose of the buffer agent is to provide or increase buffer capacity on the underside of the outer layer once intestinal fluid begins to penetrate the outer layer. Without wishing to be bound by any particular theory, the Inventors believe that the buffer agent increases the buffer capacity in the dissolving inner layer and assists the ionisation and dissolution of the polymer in the outer layer. For a given pH, the higher the buffer capacity, the faster the rate of polymer dissolution. In embodiments where there is a base in the inner layer, the buffer agent helps maintains the alkaline environment under the outer layer once intestinal fluid penetrates the outer layer.

The buffer agent(s) is present in the inner layer in an amount of from more than 20 wt % to about 60 wt %. Preferably, the buffer agent is present in the inner layer in an amount of more than about 20 wt % to about 50 wt %, or from about 25 wt % to about 40 wt %, *e.g.* about 30 wt %, based on the dry weight of the non-ionic polymer in the inner layer.

The buffer agent can be any suitable buffer agent known by the skilled person. The buffer agent may be an organic acid such as a pharmacologically acceptable non-polymeric carboxylic acid, e.g. a carboxylic acid having from 1 to 16, preferably 1 to 3, carbon atoms. Suitable carboxylic acids are disclosed in WO2008/135090A. Citric acid is an example of such a carboxylic acid. The carboxylic acids may be used in carboxylate salt form, and mixtures of carboxylic acids, carboxylate salts or both may also be used.

The buffer agent may also be an inorganic salt such as an alkali metal salt, an alkali earth metal salt, an ammonium salt, and a soluble metal salt. As metals for the soluble metal salts, manganese, iron, copper, zinc and molybdenum can be mentioned. Further preferred, the inorganic salt is selected from chloride, fluoride, bromide, iodide, phosphate, nitrate, nitrite, sulphate and borate. Phosphates such as potassium dihydrogen phosphate are preferred over other inorganic buffer salts and organic acid buffers due to their greater buffer capacity at the pH of the coating solution, for example pH 8.

In another preferred embodiment, the buffer agent is combined with a base in the form of a salt complex. The base is preferably selected from the group consisting of hydroxide bases, alkali metal bicarbonates, alkali metal carbonates, alkali metal phosphates, alkali metal citrates, or physiologically tolerated amines. More preferably, the base is a hydroxide base, and particularly preferred is sodium hydroxide.

### Optional additional layers

The inner layer is in direct contact with the surface of an isolated core, *i.e.* a core coated with an isolation layer. Thus, the formulation of the present invention has an additional isolation layer as part of the core, *i.e.* between the surface of the active core and the inner layer.

An isolation layer is particularly desirable when the composition of the core is incompatible with the delayed release coating. For example, the present invention embraces embodiments in which the inner layer provides an alkaline environment which is thought to assist in the dissolution and degradation of the outer layer. However, if the core contains a drug having acidic groups, then the inner layer may be incompatible with the core. An example of a drug having an acidic group would be 5-ASA.

Any suitable isolation layer known to the skilled person can be used. In one preferred embodiment, the isolation layer comprises a non-ionic polymer. Suitable non-ionic polymers include methylcellulose (MC); hydroxypropyl cellulose (HPC); hydroxypropyl methylcellulose (HPMC); poly(ethyleneoxide)-graft-polyvinylalcohol; polyvinylpyrollidone (PVP); polyethylene glycol (PEG); and polyvinylalcohol (PVA). HPMC or PVA is preferred. Mixtures of non-ionic polymers may also be used. A particularly preferred mixture is HPMC and PEG. The isolation layer can additionally comprise a plasticiser. Suitable plasticisers include but are not limited to polyethylene glycol, triethyl citrate (TEC), triacetin and acetyltriethyl citrate.

It is also possible to have an intermediate layer between the outer and inner layers, provided that the intermediate layer does not affect adversely the release characteristics of the formulation. However, the outer layer is usually provided in contact with the inner layer, that is to say the outer layer is usually applied directly on to the inner layer, *i.e.* there is usually no intermediate layer separating the inner and outer layers.

The formulation can optionally have an outer layer coating the delayed release composition layer of the present invention. For example, if the delayed release composition layer comprises a mixture of Eudragit^{®} L and starch, the addition of an outer layer of a pH dependent release coating material having a pH threshold of about 7, e.g. Eudragit^{®} S, may be preferable. The delayed release coating of the present invention is preferably the outer coating of the formulation. Advantageously, it has been found that no additional outer layer is required to ensure that the composition is a delayed release composition.

### The Core

The "core" is the solid body on which the coating is applied. The core may be any suitable dosage form, for example, a tablet, a pellet, a granule, a microparticle, a hard or soft capsule, or a microcapsule. In preferred embodiments, the core is a tablet or a capsule.

The core comprises the drug(s). The drug(s) may be contained within the body of the core, for example within the matrix of a tablet or a pellet, or within the contents encapsulated within a capsule. Alternatively, the drug may be in a coating applied to the core, for example where the core is a bead of edible material such as sugar, e.g. where the core is in the form of a nonpareil bead or dragée.

The core may consist of the drug(s) alone, or more usually may consist of the drug(s) and at least one pharmacologically acceptable excipient. In this connection, the core is typically a tablet or pellet and in the case of a tablet usually consists of a mixture of the drug(s) with one more excipients selected from a filler or diluent material, e.g. lactose or cellulose material such as microcrystalline cellulose; a binder, e.g. polyvinylpyrrolidone (PVP) or hydroxypropyl methylcellulose (HPMC); a disintegrant, *e.g.* croscarmellose sodium (e.g. Ac-Di-Sol^{®}) and sodium starch glycolate (e.g. Explotab^{®}); and/or a lubricant, e.g. magnesium stearate and talc. The core may be a compressed granulate comprising at least some of these materials.

The core may be uncoated or, as indicated above, the core itself may comprise a coating layer such an isolation layer onto which the inner layer and then outer layer coating are applied.

The minimum diameter of each core is typically at least about 10⁻⁴ m, usually at least about 5 × 10⁻⁴ m and, preferably, at least about 10⁻³ m. The maximum diameter is usually no more than 30 mm, typically no more than 25 mm and, preferably, no more than 20 mm. In preferred embodiments, the core has a diameter from about 0.2 mm to about 25 mm, and preferably from about 0.2 mm to about 4 mm (e.g. for pellets or mini-tablets) or from about 5 mm to about 25 mm (e.g. for certain tablets or capsules). The term "diameter" refers to the largest linear dimension through the core.

The formulation may comprise a plurality of coated cores in order to provide a single dose of the drug(s), particularly in embodiments in which the core is "small", e.g. having a diameter of less than 5 mm. Multiunit dosage forms comprising particles having a diameter of less than 3 mm are preferred.

The present invention has application in a multi-phasic drug release formulation comprising at least two pluralities of particles, e.g. coated pellets, in the same dosage form, e.g. a capsule, in which the particles of one plurality are differentiated from the particles of the or each other plurality by the coating. The coatings may differ from one plurality to the next in terms of coating thickness or composition, e.g. the ratio and/or identity of components. Multi-phasic drug release formulations would be particularly suitable for suffers of Crohn's disease affecting different regions along the intestine.

Release from formulations according to the present invention is delayed until the intestine and preferably the colon. Release from certain formulations may also be sustained. However, in preferred formulations, release is pulsatile.

The time between initial exposure to conditions suitable for drug release and the start of drug release is known as the "lag time". The "lag time" depends on a number of factors including coating thickness and composition. Formulations according to the present invention usually display a lag time in colonic conditions of at least 30 minutes. In most embodiments of the present invention, the lag time is from about 30 minutes to about 3 hours and, in preferred formulations, the lag time is preferably from about 45 minutes to about 2 hours.

A formulation is usually defined as gastric resistant if there is less than 10 wt % drug release in acidic media after 2 hours. Formulations according to the present invention typically display far less than 10 wt % drug release in acidic media and may be considered to be gastric resistant. The formulations usually display less than 1 wt % drug release in acidic media and, typically, display substantially no drug release in acidic media. When starch is combined with an acrylate film-forming material to form the coating for the core, typically less than 5% drug release occurs over 6 hours in conditions simulating the stomach and small intestine. On combination of starch with a cellulosic film forming material for the coating for the core, typically less than 10% drug release occurs over 5 hours in conditions simulating the stomach and small intestine.

The time between initial exposure to conditions suitable for drug release and complete drug release also depends on a number of factors including coating composition and the nature of the drug. In most embodiments of the present invention, this time is usually no more than 5 hours. In preferred embodiments, this time is usually no more than 4 hours.

### Method

According to a second aspect of the present invention, there is provided a method of producing a delayed release formulation according to the first aspect, said method comprising:
forming a core comprising a drug;
initially coating the core with an isolation layer coating preparation comprising a film-forming non-ionic polymer in a solvent to form an isolated core;
dissolving a film-forming non-ionic polymer that is soluble in intestinal or gastrointestinal fluid in an aqueous solvent with a buffer agent in an amount of more than 20 wt % to 60 wt %, based on the dry weight of the non-ionic polymer, to form an inner layer coating preparation having a pH of greater than pH 7;
coating the isolated core using the inner layer coating preparation to form an inner layer coated core; and
coating the inner layer coated core with an outer layer coating preparation comprising a film-forming enteric polymer having a pH threshold of pH 6 or above in a solvent system, to form an outer coated core.

The method comprises initially coating the core with an isolation layer coating preparation comprising a film-forming non-ionic polymer in a solvent to form an isolated core for coating with the inner layer coating preparation. It is preferred that the identity of the non-ionic polymer of the isolation layer coating preparation is the same as that for the non-ionic polymer of the inner layer coating preparation. Preferred film-forming non-ionic polymers are as detailed above.

In some embodiuments, the amount of buffer agent present in the aqueous solvent is not sufficient to achieve a target pH of inner coating layer preparation. In those embodiments, base is added to the inner coating layer preparation in an amount sufficient to raise the pH to the required level. It is further preferred that the amount of base added to the inner layer coating preparation is sufficient to raise the pH of the inner layer coating preparation to be in a range from pH 7.5 to pH 10, more preferably in a range from pH 7.5 to pH 8.5, and most preferably pH 8.

Preferred bases are as detailed above.

It is preferred that the core is coated with the inner layer coating preparation until the non-ionic polymer is coated on to the core in an amount from 2 mg/cm² to 5 mg/cm², e.g. 3 mg/cm², based on the dry weight of the non-ionic polymer.

The buffer agent is preferably present in the inner layer coating preparation in an amount of more than 20 wt % to 50 wt %, of from 25 wt % to 40 wt %, e.g. 30 wt %, based on the dry weight of the non-ionic polymer. Preferred buffer agents are as detailed above.

It is preferred that the outer layer coating preparation comprises a mixture of the enteric polymer and an enzymatically degradable polymer that is susceptible to attack by colonic enzymes. It is further preferred that when the enzymatically degradable polymer and the enteric polymer are present in the outer layer coating preparation, they are present in a ratio of more than 10:90 to 80:20, e.g. from 10:90 to 60:40 and preferably from 25:75 to 60:40. In some particularly preferred embodiments, the proportion is from 15:85 to 35:65, e.g. from 25:75 to 35:65 and preferably 30:70. In other particularly preferred embodiments, the proportion is from 40:60 to 60:40, *e.g.* 50:50.

Preferred enzymatically degradable polymers and enteric polymers suitable for use in the outer layer are as detailed above.

It is preferred that the inner coated core is coated with the outer layer coating preparation until the enteric polymer is coated on to the inner coated core in an amount from about 2 mg/cm² to about 10 mg/cm², *e.g.* from about 2 mg/cm² to about 8 mg/cm², or from about 4 mg/cm² to about 8 mg/cm², or from about 5 mg/cm² to about 8 mg/cm², or from about 6 mg/cm² to about 8 mg/cm², or from about 7 mg/cm² to about 8 mg/cm², *e.g.* about 7.5 mg/cm².

In embodiments in which the outer layer coating preparation comprises a mixture of the enteric polymer and an enzymatically degradable polymer, the method of preparing the outer coating preparation typically comprises the steps of:
forming an organic solution or an aqueous dispersion comprising the enteric polymer;
forming an alcoholic or an aqueous solution comprising the enzymatically degradable polymer; and
adding, preferably drop-wise, at least a portion of the alcoholic or an aqueous solution comprising the enzymatically degradable polymer to the organic solution or the aqueous dispersion comprising the enteric polymer to form the outer layer coating preparation.

In embodiments where the enzymatically degradable polymer is starch, it is preferably dispersed in at least one alcohol, preferably a C₁ to C₆ alcohol, *e.g.* methanol; ethanol; propan-1-ol; propan-2-ol; butan-1-ol; butan-2-ol; and mixtures thereof, particularly butan-1-ol alone, and then water is usually added subsequently with good agitation. The resulting aqueous dispersion is usually heated to boiling and then cooled with stirring overnight. The purpose of the alcohol(s) is to solvate the first material ready to form the aqueous dispersion. Alternatively, the material can be dispersed directly in water.

The enteric polymer is typically dissolved in at least one solvent, for instance water or an organic solvent. The organic solvent may be an alcohol, *e.g.* methanol; ethanol; propan-2-ol; methyl glycol; butyl glycol; acetone; methyl glycol acetate; and mixtures thereof such as acetone and isopropyl alcohol (*e.g.* in a ratio of about 2:3). The enteric polymer is preferably dissolved in ethanol (preferably from 85 to 98%), under high speed stirring.

The outer coating preparation is preferably formed by adding an appropriate quantity of the alcoholic or aqueous solution or dispersion comprising the enzymatically degradable polymer to the organic solution or the aqueous dispersion comprising the enteric polymer, drop-wise under fast stirring. The further excipient(s) such as a plasticiser (*e.g.* triethyl citrate (TEC)) and/or a lubricant (*e.g.* glyceryl monostearate) are usually added to the preparation while stirring.

### Different Aspects of the Disclosure

In a further aspect, there is provided a formulation according to the first aspect for use in a method of medical treatment of the human or animal body by therapy.

The core comprises at least one drug. The formulation is usually used to administer a single drug as the sole therapeutically active component. However, more than one drug may be administered in a single formulation.

The formulation of the present invention can comprise any size core. In some embodiments, the drug can be present in the core of the formulation in an amount of from about 50 mg to about 1650 mg, or from about 100 mg to about 1550 mg, or from about 150 mg to about 1500 mg, or from about 200 mg to about 1450 mg, or from about 250 mg to about 1400 mg, or from about 300 mg to about 1350 mg, or from about 350 mg to about 1300 mg, or from about 400 mg to about 1250 mg, or from about 450 mg to about 1200 mg, or from about 500 mg to about 1150 mg, or from about 550 mg to about 1100 mg, or from about 600 mg to about 1050 mg, or from about 650 mg to about 1000 mg, or from about 700 mg to about 950 mg, of from about 800 mg to about 1600 mg, or from about 850 mg to about 1600 mg, or from about 900 mg to about 1500 mg, or from about 950 mg to about 1400 mg or from about 1000 to about 1300 mg, or from about 1150 about 1200 mg. Preferably, the drug is present in the core amount selected from about 400 mg, about 800 mg, about 1200 mg, about 1500 mg, or about 1600 mg.

The formulation of the present invention is designed to administer a wide range of drugs. Suitable drugs include those drugs which are known for intestinal administration using known delayed release oral formulations. The present invention may be used to administer drugs having a local or a systemic effect.

The formulation of the present invention has particular application in the intestinal administration of a drug comprising at least one acidic group such as a carboxylic acid group. Such drugs may be acidic drugs or zwitterionic drugs. An example of such a drug is 5-aminosalicylic acid (5-ASA).

The identity of the drug(s) in the formulation obviously depends on the condition to be treated. In this connection, the formulation has particular application in the treatment of IBD (including Crohn's disease and ulcerative colitis); IBS; constipation; diarrhoea; infection; and carcinoma, particularly colon or colorectal cancer).

For the treatment or prevention of IBD, which is not claimed, the formulation may comprise at least one drug selected from the group consisting of anti-inflammatory agents (*e.g.* 5-ASA, 4-ASA, sulphasalazine and balsalazide); non-steroidal anti-inflammatory agents (*e.g.* ibuprofen and diclofenac); steroids (*e.g.* prednisolone; budesonide or fluticasone); immunosuppressants (*e.g.* azathioprine; cyclosporin; and methotrexate); antibiotics; and biological agents including peptides, proteins and antibody fragments. Suitable examples of biological agents include alkaline phosphatase and anti-TNF antibodies such as infliximab, adalimumab, certulizumab pegol, golimumab and ustekinumab.

For the treatment or prevention of cancer, which is not claimed, the formulation may comprise at least one antineoplastic agent. Suitable antineoplastic agents include fluorouracil; methotrexate; dactinomycin; bleomycin; etoposide; taxol; vincristine; doxorubicin; cisplatin; daunorubicin; VP-16; raltitrexed; oxaliplatin; and pharmacologically acceptable derivatives and salts thereof. For the prevention of colon cancer or colorectal cancer, primarily in patients suffering from colitis, which is not claimed, the formulation may comprise the anti-inflammatory agents 5-ASA, sulindac, celecoxib and/or eflornithine (DFMO).

For the treatment or prevention of IBS, constipation, diarrhoea or infection, which is not claimed, the formulation may comprise at least one active agent suitable for the treatment or prevention of these conditions.

Pharmacologically acceptable derivatives and/or salts of the drugs may also be used in the formulation. An example of a suitable salt of prednisolone is methyl prednisolone sodium succinate. A further example is fluticasone propionate.

The present invention has particular application in either the treatment of IBD (particularly, ulcerative colitis) or the prevention of colon cancer or colorectal cancer (primarily in colitis patients), both using 5-ASA. It also has application as a portal of entry of drugs into the systemic circulation via the colon. This is particularly advantageous for peptide and protein drugs which are unstable in the upper gastrointestinal tract. The present invention may also be utilised for the purpose of chronotherapy.

In a third aspect of the disclosure, which is not claimed, there is provided a method of targeting a drug to the colon comprising administering to a patient a formulation as defined above.

In a fourth aspect of the disclosure, which is not claimed, there is provided the use of a formulation as defined above in the manufacture of a medicament for the treatment or prevention of IBD (particularly ulcerative colitis); IBS; constipation; diarrhoea; infection; and cancer.

There is also provided the use of at least one drug selected from anti-inflammatory agents and steroids in the manufacture of a medicament comprising a formulation as defined above for use in the treatment of IBD. In addition, there is also provided the use of at least one antineoplastic agent in the manufacture of a medicament comprising a formulation as defined above for use in the treatment of carcinoma. Further, there is also provided use of 5-ASA in the manufacture of a medicament comprising a formulation as defined above for use in the prevention of colon cancer or colorectal cancer.

According to a fifth aspect of the present disclosure, which is not claimed, there is provided a method of medical treatment or prevention of IBD or carcinoma comprises administering to a patient a therapeutic amount of a formulation as defined above.

The formulation will typically comprise a therapeutically effective amount of the or each drug which may be from about 0.01 wt % to about 99 wt %, based on the total weight of the formulation. The actual dosage would be determined by the skilled person using his common general knowledge. However, by way of example, "low" dose formulations typically comprise no more than about 20 wt % of the drug, and preferably comprise from about 1 wt % to about 10 wt %, *e.g.* about 5 wt %, of the drug. "High" dose formulations typically comprise at least 40 wt % of the drug, and preferably from about 45 wt % to about 85 wt %, *e.g.* about 50 wt % or about 80 wt %.

### Food effect

The drug release profile from a conventional delayed release dosage form is often dependent on the state of the stomach, *i.e.* whether the stomach is in the "fed" state or the "fasted" state. In brief, the "fed state" leads to enhanced gastric residence time which can influence t_{lag}, *i.e.* the time before initial release of the drug from the dosage form. In addition, fast *in vivo* dissolution after leaving the stomach may lead to an increase in Cₘₐₓ, or the peak blood plasma concentration for the drug.

The dependency of drug release on the state of the stomach is known colloquially as the "food effect" and is the reason why conventional dosage forms are often to be administered either on an empty stomach, or with or just after food. Clearly, a significant food effect is undesirable as dictates when an oral dosage form may be administered which could have an adverse effect on patient safety and efficacy due to premature drug release.

The fasted and fed states can be simulated *in vitro* by exposing dosage forms initially to either 0.1 N HCl for 2 hours (fasted state) or to Fed State Simulated Gastric Fluid (FeSSGF), for example, at pH 5 for 4 hours. After the simulated fasted or fed states, the tablets are further exposed to Krebs buffer at pH 7.4 for at least 4 hours which to simulates the conditions of the ileo-colonic region. Exposing the tablets for longer than 4 hours, *e.g.* for 10 hours in Hanks buffer at pH 6.8 as in the examples discussed below, can provide an indication of the "robustness" of the tablets in simulated upper small intestinal conditions.

An example of FeSSGF is described in Jantratid et al (2008) "Dissolution media simulating conditions in the proximal human gastrointestinal tract: An update." (Pharm. Res. 25(7): 1663-1676). In brief, this example of FeSSGF is composed of a mixture (50:50) of milk and acetic acid/sodium acetate buffer and sodium chloride.

By way of example, the Inventors have observed that coated 800 mg 5-ASA tablets (coated with single coating of Eudragit^{®} S) demonstrate shorter t_{lag} when exposed *in vitro* to this simulated fed state conditions compared to the simulated fasted state conditions. Earlier initial release of 5-ASA may result in absorption of the drug in the small intestine which could lead to an increase in systemic side effects. A similar effect is also observed both *in vitro* and *in vivo* for Lialda^{®}/Mezavant^{®}, a 1200 mg 5-ASA tablet formulation from Cosmo Pharmaceuticals/Shire intended for site specific colonic release of 5-ASA.

### Examples

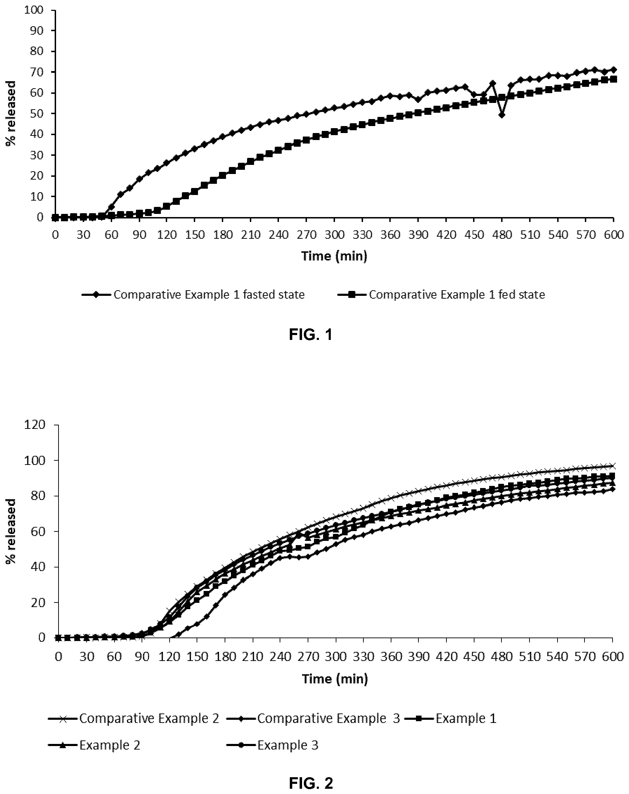

A number of preferred embodiments of the present invention will now be described with reference to the drawings, in which:-
FIG. 1 is a graph comparing drug release as a function of time from coated 5-ASA tablets according to Comparative Example 1, when exposed to (a) FaSSGF for 2 hours and then Kreb's buffer (pH 7.4) for 10 hours; and (b) FeSSGF for 4 hours then Krebs buffer (pH 7.4) for 10 hours. Only data in pH 7.4 Krebs buffer is represented;
FIG. 2 is a graph comparing drug release as a function of time from coated 5-ASA tablets according to according to Comparative Examples 2 and 3 and Examples 1 to 3 when exposed to FaSSGF for 2 hours and then Kreb's buffer (pH 7.4) for 10 hours. Only data in pH 7.4 Krebs buffer is represented;

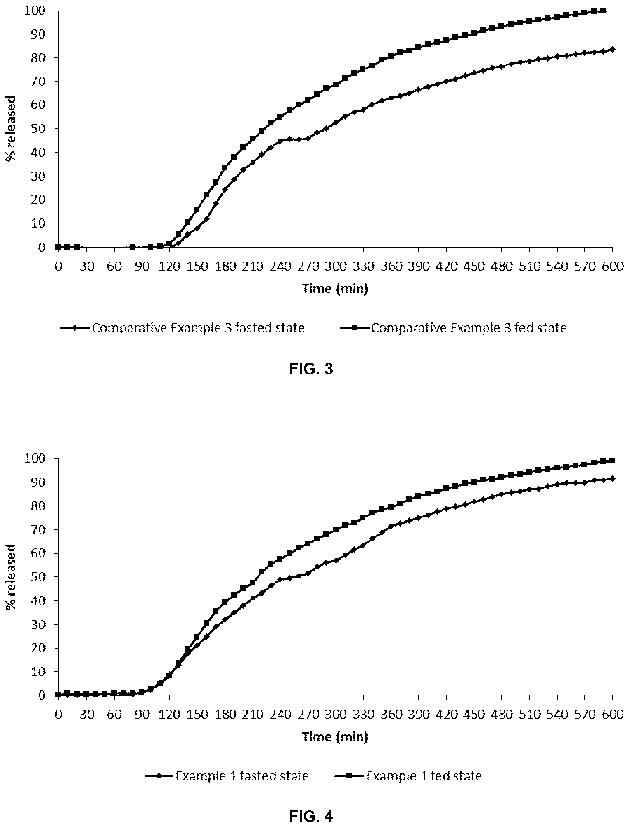

FIG. 3 is a graph comparing drug release as a function of time from coated 5-ASA tablets according to according to Comparative Example 3, when exposed to (a) FaSSGF for 2 hours and then Kreb's buffer (pH 7.4) for 10 hours; and (b) FeSSGF for 4 hours then Krebs buffer (pH 7.4) for 10 hours. Only data in pH 7.4 Krebs buffer is represented;
FIG. 4 is a graph comparing drug release as a function of time from coated 5-ASA tablets according to Example 1, when exposed to (a) FaSSGF for 2 hours then Krebs buffer (pH 7.4) for 10 hours; and (b) FeSSGF for 4 hours then Krebs buffer (pH 7.4) for 10 hours. Only data in pH 7.4 Krebs buffer is represented;

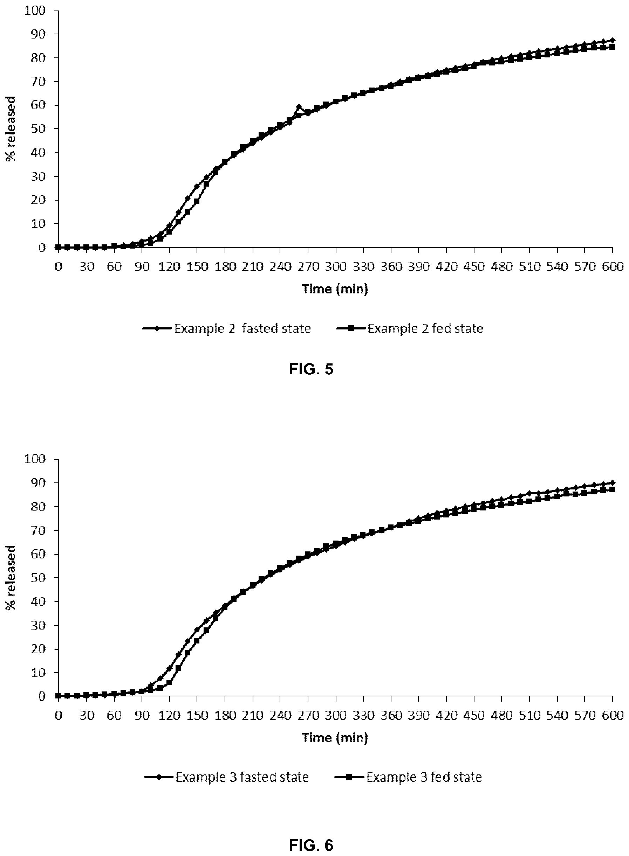

FIG. 5 is a graph comparing drug release as a function of time from coated 5-ASA tablets according to Example 2, when exposed to (a) FaSSGF for 2 hours then Krebs buffer (pH 7.4) for 10 hours; and (b) FeSSGF for 4 hours then Krebs buffer (pH 7.4) for 10 hours. Only data in pH 7.4 Krebs buffer is represented;
FIG. 6 is a graph comparing drug release as a function of time from coated 5-ASA tablets according to Example 3, when exposed to (a) FaSSGF for 2 hours then Krebs buffer (pH 7.4) for 10 hours; and (b) FeSSGF for 4 hours then Krebs buffer (pH 7.4) for 10 hours. Only data in pH 7.4 Krebs buffer is represented;

### Materials

Eudragit^{®} S 100, was purchased from Evonik GmbH, Darmstadt, Germany. Maize starch (Eurylon^{®} 6) was purchased from Roquette, Lestrem, France. Polysorbate 80 (Tween^{®} 80), butan-1-ol, triethyl citrate (TEC), ethanol 95%, butanol, potassium phosphate monobasic (KH₂PO₄), sodium diphosphate dibasic dihydrate (Na₂HPO₄•2H₂O), and sodium hydroxide were all purchased from Sigma-Aldrich, Buchs, Switzerland. HPMC (Pharmacoat^{®} 603) was purchased from Shin-Etsu. Opadry^{®} AMB was purchased from Colorcon. Glyceryl monostearate (GMS) was purchased from Cognis. Polyethylene glycol (PEG 6000) was purchased from Aldrich. Iron oxide red and iron oxide yellow (Sicovit) were purchased from BASF.

### Tablet cores

Tablet cores containing 800 mg 5-ASA (Examples 1 to 3 and Comparative Examples 2 and 3 and 1200 mg 5-ASA (Comparative Example 1) were provided.

The tablet cores of Comparative Examples 1 to 3 and Example 1 were coated with an isolation layer of hydroxypropyl methylcellulose (HPMC) whereas the tablet cores of Examples 2 and 3 were coated with an isolation layer of Opadry^{®} AMB, a polyvinyl alcohol (PVA)-based coating material.

### Preparation of coated tablet cores

COMPARATIVE EXAMPLES 1 and 2 (5-ASA tablet cores coated with an HPMC isolation layer/inner layer of neutralized Eudragit^{®} S 100 with buffer and base/outer layer of a 50:50 (Comparative Example 1) or 70:30 (Comparative Example 2) mixture of Eudragit^{®} S 100 and high amylose starch)

### Isolation layer

The isolation layer was applied from an aqueous mixture of HPMC and 20% PEG 6000 in the following amounts:

**Table 1**

| **Component** | **mg/cm²** |
|---|---|
| HPMC | 3 |
| PEG 6000 | 0.6 |

The HPMC was dissolved in water under magnetic stirring and then the PEG 6000 was added to form an isolation layer coating preparation.

The isolation layer coating preparation was sprayed on to the 5-ASA cores using a pan coater having a 0.8 L drum on a batch size of 400 g until the coating amount of HPMC reached 3 mg polymer/cm² to form isolation layer coated cores. The spray coating parameters were as follows:

**Table 2**

| | |
|---|---|
| Drum speed (rpm) | 10-15 |
| Nozzle diameter (mm) | 0.8 |
| Spray rate (g/min) | 3-5.2 |
| Spray pressure (bar) | 0.7 |
| Pattern pressure (bar) | 1.0 |
| Air flow (m³/h) | 30 |
| Inlet air temperature (°C) | 65-70 |
| Outlet air temperature (°C) | 40-43 |
| Product temperature (°C) | 33-34 |

### Inner layer

The inner layer was applied from an aqueous preparation of Eudragit^{®} S 100, where the pH was adjusted to pH 8. The composition of the inner layer also included 70% TEC (based on dry polymer weight), 1% KH₂PO₄ (based on dry polymer weight), 10% GMS (based on dry polymer weight) and 40% polysorbate 80 (based on GMS weight). The pH was adjusted using 1M NaOH until pH 8 was obtained.

**Table 3**

| | **Comparative Example 1** | **Comparative Example 2** |
|---|---|---|
| | mg/cm² | |
| Eudragit^{®} S | 5 | 5 |
| KH₂PO₄ | 0.05 | 0.05 |
| Glyceryl monostearate | 0.5 | 0.5 |
| Polysorbate 80 | 0.2 | 0.2 |
| Triethyl citrate | 3.5 | 3.5 |
| 1M NaOH | As required to reach pH 8 | |

The inner layer coating preparation was prepared by dissolving the required amounts of KH₂PO₄ and TEC in distilled water, followed by dispersion of the Eudragit^{®} S 100 under mechanical agitation. The pH was then adjusted to pH 8 with 1M NaOH and mixed for 1 h to form a neutralised Eudragit^{®} S solution.

A GMS emulsion was prepared at a concentration of 10% w/w. Polysorbate 80 (40% based on GMS weight) was dissolved in distilled water followed by dispersion of GMS. This preparation was then heated to 75°C for 15 minutes under strong magnetic stirring in order to form an emulsion. The emulsion was cooled to room temperature under stirring.

The GMS emulsion was added to the neutralised Eudragit^{®} S solution and the final inner layer coating was sprayed onto isolation layer coated tablets using a perforated pan coater until the coating amount of Eudragit^{®} S 100 reached 5 mg polymer/cm² to produce inner layer coated cores. The spray coating parameters were as follows:

**Table 4**

| | **Comparative Example 1** | **Comparative Example 2** |
|---|---|---|
| Drum speed (rpm) | 12 - 16 | 12 |
| Nozzle diameter (mm) | 0.8 | 0.8 |
| Spray rate (g/min) | 3.1 | 6.8 - 7.2 |
| Spray pressure (bar) | 0.4 | 0.7 |
| Pattern pressure (bar) | 0.5 | 1.0 |
| Air flow (m³/h) | 30 | 30 |
| Inlet air temperature (°C) | 58 - 65 | 70 |
| Outlet air temperature (°C) | 37.6 - 38.0 | 34.1 - 37.8 |
| Product temperature (°C) | 24.5 - 25.5 | 25.5 - 31.5 |

### Outer layer

The inner layer coated tablet cores were coated with an outer coating formed of 50% Eudragit^{®} S 100 and 50% high amylose starch (Comparative Example 1) or with an outer layer formed of 70% Eudragit^{®} S 100 and 30% high amylose starch (Comparative Example 2).

The outer layer coating was applied from a mixture of an aqueous starch dispersion and an ethanolic Eudragit^{®} S 100 solution in the following amounts (based on Eudragit^{®} S 100 dry polymer weight):

**Table 5**

| | **Comparative Example 1** | **Comparative Example 2** |
|---|---|---|
| | mg/cm² | |
| Starch (raw) | 5.71 | 2.45 |
| Glyceryl monostearate | 0.5 | 0.36 |
| Polysorbate 80 | 0.2 | 0.14 |
| Iron Oxide yellow | 0.11 | 0.11 |
| Iron Oxide red | 0.66 | 0.66 |
| Eudragit^{®} S 100 | 5 | 5.00 |
| Triethyl citrate | 2 | 2 |

The aqueous starch dispersion was prepared by dispersing high amylose maize starch, (Eurylon^{®} 6 also known as Amylo N-400) into butan-1-ol, followed by water, under magnetic stirring. The resulting dispersion was heated to boiling and then cooled under stirring overnight.

The Eudragit^{®} S 100 solution was prepared by dispersing Eudragit^{®} S 100 in 96% ethanol under high speed stirring.

The aqueous starch dispersion was added dropwise to the Eudragit^{®} S 100 solution under stirring to obtain a ratio of Eudragit^{®} S 100:starch of 50:50 (Comparative Example 1) or 70:30 (Comparative Example 2). The mixture was stirred for 1 hour and triethyl citrate and a GMS emulsion (previously prepared with Polysorbate 80) were added and mixed for further 30 minutes. A suspension of iron oxide red and iron oxide yellow was added and the mixture was stirred for a further 10 minutes.

The GMS emulsion was prepared at a concentration of 5% w/w. Polysorbate 80 (Tween^{®}, 40% based on GMS weight) was dissolved in distilled water followed by dispersion of the GMS. The dispersion was heated at 75 °C for 15 minutes under strong magnetic stirring in order to form an emulsion. The emulsion was cooled at room temperature under stirring. The pigment suspension was formed by suspending red and yellow iron oxide pigments in 96% ethanol for 10 minutes under homogenization.

The final outer layer coating preparation was sprayed on to the inner layer coated cores using the same pan coater as used to apply the isolation layer having a 0.8 L drum on a batch size of 400 g until the coating amount of Eudragit^{®} S 100 reached 5 mg polymer/cm². The spray coating parameters were as follows:

**Table 6**

| | **Comparative Example 1** | **Comparative Example 2** |
|---|---|---|
| Drum speed (rpm) | 12 | 12 |
| Nozzle diameter (mm) | 1.0 | 0.8 |
| Spray rate (g/min) | 2.95 | 3.2 |
| Spray pressure (bar) | 0.4 | 0.4 |
| Pattern pressure (bar) | 0.5 | 0.5 |
| Air flow (m³/h) | 40 | 40 |
| Inlet air temperature (°C) | 53 - 55 | 52 - 55 |
| Outlet air temperature (°C) | 40.5 - 41.6 | 40.9 - 42.6 |
| Product temperature (°C) | 34.5 - 36.5 | 34.5 - 36.5 |

COMPARATIVE EXAMPLE 3 and EXAMPLE 1 (5-ASA tablet cores coated with a HPMC isolation layer/inner layer of HPMC with 10 wt % buffer salt (Comparative Example 3) or 30 wt % buffer salt (Example 1) and a base/outer layer of a 70:30 mixture of Eudragit^{®} S 100 and high amylose starch).

The isolation layer was applied as described for Comparative Example 1 and 2.

The inner layer was applied from a mixture of HPMC, 20% PEG 6000 (based on HPMC dry weight), and a KH₂PO₄ buffer agent in the following amounts:

**Table 7**

| | **Comparative Example 1** | **Example 1** |
|---|---|---|
| | mg/cm² | |
| HPMC | 3 | |
| KH₂PO₄ | 0.3 | 0.9 |
| PEG 6000 | 0.6 | |
| 1M NaOH | As required to reach pH 8 | |

The inner layer coating preparation was prepared by dissolving the required amount of KH₂PO₄ and PEG 6000 in water under magnetic stirring. The HPMC was added slowly and allowed to stir until complete dissolution was observed. The pH of the solution was adjusted to pH 8 by adding aliquots of 1M NaOH.

The inner layer coating preparation was sprayed on to the isolation layer coated cores using the same pan coater as for the isolation layer until the coating amount of HPMC reached 3 mg polymer/cm² to form inner layer coated cores. The spray coating parameters were as follows:

**Table 8**

| | **Comparative Example 3 and Example 1** |
|---|---|
| Drum speed (rpm) | 10 - 12 |
| Nozzle diameter (mm) | 0.8 |
| Spray rate (g/min) | 2.5 |
| Spray pressure (bar) | 0.6 |
| Pattern pressure (bar) | 0.8 |
| Air flow (m³/h) | 30 |
| Inlet air temperature (°C) | 62 - 70 |
| Outlet air temperature (°C) | 40.6 - 40.9 |
| Product temperature (°C) | 30.5 - 31 |

### Outer layer

The inner layer coated tablet cores were coated with an outer coating formed of 70% Eudragit^{®} S 100 and 30% high amylose starch.

The outer coating was applied from a mixture of an aqueous starch dispersion and an ethanolic Eudragit^{®} S 100 solution in the following amounts (based on Eudragit^{®} S 100 dry polymer weight):

**Table 9**

| | **Comparative Example 3 and Example 1** |
|---|---|
| | mg/cm² |
| Starch (raw) | 2.45 |
| Glyceryl monostearate | 0.36 |
| Polysorbate 80 | 0.14 |
| Iron Oxide yellow | 0.11 |
| Iron Oxide red | 0.66 |
| Eudragit^{®} S 100 | 5 |
| Triethyl citrate | 1.43 |

The aqueous starch dispersion was prepared by dispersing high amylose maize starch, (Eurylon^{®} 6 also known as Amylo N-400) into butan-1-ol, followed by water, under magnetic stirring. The resulting dispersion was heated to boiling and then cooled under stirring overnight.

The Eudragit^{®} S 100 solution was prepared by dispersing Eudragit^{®} S 100 in 96% ethanol under high speed stirring.

The aqueous starch dispersion was added dropwise to the Eudragit^{®} S 100 solution under stirring to obtain a ratio of Eudragit^{®} S 100:starch of 70:30 (Example 1 and 2) or 50:50 (Example 3). The mixture was stirred for 1 hour and triethyl citrate and a GMS emulsion (previously prepared with Polysorbate 80) were added and mixed for further 30 minutes. A suspension of iron oxide red and iron oxide yellow was added and the mixture was stirred for a further 10 minutes.

The GMS emulsion was prepared at a concentration of 5% w/w. Polysorbate 80 (Tween^{®}, 40% based on GMS weight) was dissolved in distilled water followed by dispersion of the GMS. The dispersion was heated at 75 °C for 15 minutes under strong magnetic stirring in order to form an emulsion. The emulsion was cooled at room temperature under stirring.

The pigment suspension was formed by suspending red and yellow iron oxide pigments in 96% ethanol for 10 minutes under homogenization.

The final outer layer coating preparation was sprayed on to the inner layer coated cores using the same pan coater as used to apply the isolation layer having a 0.8 L drum on a batch size of 400 g until the coating amount of Eudragit^{®} S 100 reached 5 mg polymer/cm². The spray coating parameters were as follows:

**Table 10**

| | |
|---|---|
| Drum speed (rpm) | 12 |
| Nozzle diameter (mm) | 0.8 |
| Spray rate (g/min) | 3.8 |
| Spray pressure (bar) | 0.4 |
| Pattern pressure (bar) | 0.5 |
| Air flow (m³/h) | 40 |
| Inlet air temperature (°C) | 55-57 |
| Outlet air temperature (°C) | 41-43 |
| Product temperature (°C) | 32.5-33 |

EXAMPLE 2 and EXAMPLE 3 (5-ASA tablet cores coated with a PVA-based isolation layer/inner layer of HPMC with a buffer and a base/outer layer of a 70:30 mixture of Eudragit^{®} S 100 and high amylose starch)

### Isolation layer

The isolation layer was applied from an aqueous dispersion of Opadry^{®} AMB at 3.1 mg/cm² (total solids).

Opadry^{®} AMB is a fully formulated coating system based on polyvinyl alcohol (PVA). Opadry^{®} AMB was diluted with purified water under magnetic stirring for 30 minutes to prepare a isolation layer coating preparation.

The isolation layer coating preparation was sprayed onto the 5-ASA cores using a pan coater having a 0.8 L drum on a batch size of 400 g until the coating amount of Opadry^{®} AMB reached the target amount to form isolation layer coated cores. The spray coating parameters were the same as for Comparative Example 3 and Examples 1.

### Inner layer

The inner layer was applied from a mixture of HPMC, 20% PEG 6000 (based on HPMC dry weight), and a KH₂PO₄ buffer agent in the following amounts:

**Table 11**

| | **Example 2** | **Example 3** |
|---|---|---|
| | mg/cm² | |
| HPMC | 3 | |
| KH₂PO₄ | 0.9 | 1.5 |
| PEG 6000 | 0.6 | |
| 1M NaOH | As required to reach pH 8 | |

The inner layer was prepared and applied to the isolation layer coated cores according to Comparative Example 3 and Example 1.

### Outer layer

The outer coating was prepared and applied according to Comparative Example 3 and Example 1.

### Results

### Drug release test #1 - Simulated fasted state then Krebs buffer at pH 7.4

*In vitro* dissolution studies were performed on a USP type II apparatus using a paddle speed of 50 rpm and a media temperature of 37 ± 0.5 °C.

To simulate the "fasted" state, the tablets were first tested in 0.1 M HCl for 2 hours (FaSSGF) followed by 10 hours in Krebs buffer (pH 7.4).

### Drug release test #2 - Simulated fed state then Krebs buffer at pH 7.4

*In vitro* dissolution studies were performed on a USP type II apparatus using a paddle speed of 50 rpm and a media temperature of 37 ± 0.5 °C.

To simulate the "fed" state, the tablets were first tested in Fed State Simulated Gastric Fluid (FeSSGF) at pH 5.0 for 4 hours followed by 10 hours in Krebs buffer (pH 7.4). The FeSSGF was as described in Jantrid *et al* (2008) *supra.*

The results are shown in Figures 1 to 6.

The tablets according to Comparative Example 1 showed a delay in release in simulated fed state gastric conditions in comparison to fasted simulated gastric conditions (FIG.1).

The tablets according to Examples 1 to 3 showed similar properties to the tablets of Comparative Example 2 after exposure to 0.1 N HCl for 2 hours. In particular, there was no release of 5-ASA from any of the tablets tested in the 2 hours that the tablets were exposed to simulated gastric conditions. However, it should be noted that, once the tablets were exposed to pH 7.4 (drug release test #2), rapid release of 5-ASA was observed (FIG.2). It should also be noted that an inner layer containing only 10 wt % buffer salt showed the slowest latgtime in simulated fasted state gastric conditions (Comparative Example 3, FIG.2). The data in FIG.2 also demonstate that replacing the neutralized Eudragit^{®} S 100 inner layer of Comparative Example 2 with an inner layer of HPMC containing more than 10 wt % buffer salt (Examples 1 to 3) was equally effective in promoting a fast drug release.

Tablets according to Examples 1 to 3 having an inner layer of HPMC and between 30 wt % and 50 wt % KH₂PO₄ buffer salt (based on dry polymer weight) showed no delay in release of 5-ASA in pH 7.4 Krebs buffer after exposure to simulated fed gastric fluid in comparison to release after expxosure to fasted simulated gastric fluid (FIG.4 to FIG.6).

Tablets according to Comparative Example 3, having a middle layer of HPMC containing 10 wt % buffer salt (based on dry polymer weight) showed a slower dissolution rate in pH 7.4 Krebs buffer after pre-exposure to simulated fed gastric fluid (FIG.3) in comparison to tablets according to Examples 1 to 3 having between 30 wt % and 50 wt % buffer salt in the inner layer (FIG.4 to FIG.6).

Without being bound by theory, it is thought that where the outer layer of the formulation is permeable to the gastric fluid, such as in fed state simulated gastric fluid (FeSSGF), thereby allowing access to the inner layer, modification of the inner layer can occur which can affect the drug release. The buffer salt content in the inner layer made from a neutral polymer, such as HPMC, can contribute to maintaining a high buffer capacity at the interface between the coating layer and the tablet core, decreasing or avoiding the impact of the fed state gastric fluid in delaying drug release in pH 7.4 Krebs buffer which resembles the luminal electrolyte composition of the distal small intestine.

It will be appreciated that the invention is not restricted to the details described above with reference to the preferred embodiments but that numerous modifications and variations can be made within the scope of the following claims.

## Claims

1. A delayed release drug formulation for oral administration to deliver a drug to the colon of a subject, said formulation comprising:
a core, a coating for the core and an isolation layer between the core and the coating, the core comprising a drug, the coating comprising an outer layer and an inner layer,
wherein the outer layer comprises a film-forming enteric polymer having a pH threshold at pH 6 or above, and
wherein the inner layer comprises a film-forming non-ionic polymer that is soluble in intestinal or gastrointestinal fluid, and a buffer agent in an amount of more than 20 wt % to 60 wt %, based on the dry weight of the non-ionic polymer.

2. A delayed release drug formulation as claimed in Claim 1, wherein the non-ionic polymer is a non-ionic cellulose-based polymer, preferably wherein the non-ionic polymer is selected from methylcellulose (MC), hydroxypropylcellulose (HPC) and hydroxypropylmethylcellulose (HPMC).

3. A delayed release drug formulation as claimed in any of the preceding claims, wherein the non-ionic polymer is HPMC.

4. A delayed release drug formulation as claimed in Claim 1, wherein the non-ionic polymer is a non-ionic acrylate polymer or a polyvinyl-based polymer.

5. A delayed release drug formulation as claimed in any of the preceding claims, wherein the non-ionic polymer is present in the inner layer in an amount from 2 mg/cm² to 5 mg/cm² based on the dry weight of the non-ionic polymer.

6. A delayed release drug formulation as claimed in any of the preceding claims, wherein the buffer agent is present in the inner layer in an amount from 25 wt % to 60 wt % based on the dry weight of the non-ionic polymer in the inner layer.

7. A delayed release drug formulation as claimed in any of the preceding claims, wherein the buffer agent is selected from the group consisting of a carboxylic acid having from 1 to 16 carbon atoms, an alkali metal salt, an alkali earth metal salt, an ammonium salt and a soluble metal salt.

8. A delayed release drug formulation as claimed in any of the preceding claims, wherein the buffer agent is a phosphate salt, preferably wherein the buffer agent is potassium dihydrogen phosphate.

9. A delayed release drug formulation as claimed in any of the preceding claims, wherein the buffer agent is combined with a base.

10. A delayed release drug formulation as claimed in Claim 9, wherein the base is selected from the group consisting of hydroxide bases, alkali metal bicarbonates, alkali metal carbonates, alkali metal phosphates, alkali metal citrates, or physiologically tolerated amines.

11. A delayed release drug formulation as claimed in Claim 9 or Claim 10, wherein the base is a hydroxide base, preferably wherein the base is sodium hydroxide.

12. A delayed release drug formulation as claimed in any of the preceding claims wherein the outer layer comprises a mixture of the enteric polymer and an enzymatically degradable polymer that is degraded by colonic enzymes.

13. A delayed release drug formulation as claimed in Claim 12, wherein the enzymatically degradable polymer and the enteric polymer are present in the outer coating in a ratio of more than 10:90.

14. A delayed release drug formulation as claimed in Claim 12 or Claim 13, wherein the enteric polymer is present in the outer layer in an amount from 3 to 10 mg/cm², based on the dry weight of the enteric polymer.

15. A delayed release drug formulation as claimed in any of the preceding claims, wherein the isolation layer comprises a film-forming non-ionic polymer, preferably the same film-forming non-ionic polymer as present in the inner layer.

16. A method of producing a delayed release drug formulation for oral administration to deliver a drug to the colon as claimed in Claim 1, said method comprising:
forming a core comprising a drug;
initially coating the core with an isolation layer coating preparation comprising a film-forming non-ionic polymer ina solvent to form an isolated core;
dissolving a film-forming non-ionic polymer that is soluble in intestinal or gastrointestinal fluid in an aqueous solvent with a buffer agent in an amount of more than 20 wt % to 60 wt %, based on the dry weight of the non-ionic polymer, to form an inner layer coating preparation having a pH of greater than pH 7;
coating the isolated core using the inner layer coating preparation to form an inner layer coated core; and
coating the inner layer coated core with an outer layer coating preparation comprising a film-forming enteric polymer having a pH threshold of pH 6 or above in a solvent system, to form an outer coated core.

17. A method as claimed in Claim 16, wherein the method comprises adding base to the inner coating layer preparation in an amount sufficient to raise the pH to the required level.

18. A method as claimed in Claim 17, wherein the amount of base added to the inner layer coating preparation is sufficient to raise the pH of the inner layer coating preparation to be in a range from pH 7.5 to pH 10.

19. A method as claimed in Claim 17 or Claim 18, wherein the base is selected from the group consisting of hydroxide bases, alkali metal bicarbonates, alkali metal carbonates, alkali metal phosphates, alkali metal citrates, or physiologically tolerated amines.

20. A method as claimed in any of Claims 17 to 19, wherein the base is a hydroxide base, preferably wherein the base is sodium hydroxide.

21. A method as claimed in any of Claims 16 to 20, wherein the non-ionic polymer is a non-ionic cellulose-based polymer, preferably wherein the non-ionic polymer is selected from methylcellulose (MC), hydroxypropylcellulose (HPC) and hydroxypropylmethylcellulose (HPMC).

22. A method as claimed in Claim 21, wherein the non-ionic polymer is HPMC.

23. A method as claimed in any of Claims 16 to 22, wherein the core is coated with the inner layer coating preparation until the non-ionic polymer is coated on to the core in an amount from 2 to 5 mg/cm² based on the dry weight of the non-ionic polymer.

24. A method as claimed in any of Claims 16 to 23, wherein the buffer agent is present in the inner layer coating preparation in an amount of from 25 wt % to 60 wt % based on the dry weight of the non-ionic polymer.

25. A method as claimed in any of Claims 16 to 24, wherein the buffer agent is selected from the group consisting of a carboxylic acid having from 1 to 16 carbon atoms, an alkali metal salt, an alkali earth metal salt, an ammonium salt and a soluble metal salt.

26. A method as claimed in any of Claims 16 to 25, wherein the buffer agent is a phosphate salt, preferably wherein the buffer agent is potassium dihydrogen phosphate.

27. A method as claimed in any of Claims 16 to 26, wherein the outer layer coating preparation comprises a mixture of the enteric polymer and an enzymatically degradable polymer that is susceptible to attack by colonic enzymes.

28. A method as claimed in Claim 27, wherein the enzymatically degradable polymer and the enteric polymer are present in the outer layer coating preparation in a ratio of more than 10:90.

29. A method as claimed in Claim 27 or Claim 28, wherein the inner coated core is coated with the outer layer coating preparation until the enteric polymer is coated on to the inner coated core in an amount from 3 to 10 mg/cm² based on the dry weight of the enteric polymer.

30. A method as claimed in any of Claims 16 to 29, wherein the identity of the non-ionic polymer of the isolation layer coating preparation is the same as that for the non-ionic polymer of the inner layer coating preparation.

## Patentansprüche

1. Wirkstoffformulierung mit verzögerter Freisetzung zur oralen Verabreichung, um einen Wirkstoff an den Dickdarm eines Probanden abzugeben, wobei die Formulierung umfasst:
einen Kern, einen Überzug für den Kern und eine Isolationsschicht zwischen dem Kern und dem Überzug, wobei der Kem einen Wirkstoff umfasst, wobei der Überzug eine Außenschicht und eine Innenschicht umfasst,
wobei die Außenschicht ein filmbildendes magensaftresistentes Polymer mit einem pH-Schwellenwert bei pH 6 oder höher umfasst und
wobei die Innenschicht ein filmbildendes nichtionisches Polymer, das in Darm- oder Gastrointestinalflüssigkeit löslich ist, und einen Puffer in einer Menge von mehr als 20 Gew.-% bis 60 Gew.-%, bezogen auf das Trockengewicht des nichtionischen Polymers, umfasst.

2. Wirkstoffformulierung mit verzögerter Freisetzung nach Anspruch 1, wobei das nichtionische Polymer ein nichtionisches Polymer auf Cellulosebasis ist, vorzugsweise wobei das nichtionische Polymer aus Methylcellulose (MC), Hydroxypropylcellulose (HPC) und Hydroxypropylmethylcellulose (HPMC) ausgewählt ist.

3. Wirkstoffformulierung mit verzögerter Freisetzung nach einem der vorhergehenden Ansprüche, wobei das nichtionische Polymer HPMC ist.

4. Wirkstoffformulierung mit verzögerter Freisetzung nach Anspruch 1, wobei das nichtionische Polymer ein nichtionisches Acrylatpolymer oder ein Polymer auf Polyvinylbasis ist.

5. Wirkstoffformulierung mit verzögerter Freisetzung nach einem der vorhergehenden Ansprüche, wobei das nichtionische Polymer in der Innenschicht in einer Menge von 2 mg/cm² bis 5 mg/cm², bezogen auf das Trockengewicht des nichtionischen Polymers, vorliegt.

6. Wirkstoffformulierung mit verzögerter Freisetzung nach einem der vorhergehenden Ansprüche, wobei der Puffer in der Innenschicht in einer Menge von 25 Gew.-% bis 60 Gew.-%, bezogen auf das Trockengewicht des nichtionischen Polymers in der Innenschicht, vorliegt.

7. Wirkstoffformulierung mit verzögerter Freisetzung nach einem der vorhergehenden Ansprüche, wobei der Puffer aus der Gruppe bestehend aus einer Carbonsäure mit von 1 bis 16 Kohlenstoffatomen, einem Alkalimetallsalz, einem Erdalkalimetallsalz, einem Ammoniumsalz und einem löslichen Metallsalz ausgewählt ist.

8. Wirkstoffformulierung mit verzögerter Freisetzung nach einem der vorhergehenden Ansprüche, wobei der Puffer ein Phosphatsalz ist, vorzugsweise wobei der Puffer Kaliumdihydrogenphosphat ist.

9. Wirkstoffformulierung mit verzögerter Freisetzung nach einem der vorhergehenden Ansprüche, wobei der Puffer mit einer Base kombiniert ist.

10. Wirkstoffformulierung mit verzögerter Freisetzung nach Anspruch 9, wobei die Base aus der Gruppe bestehend aus Hydroxidbasen, Alkalimetallbicarbonaten, Alkalimetallcarbonaten, Alkalimetallphosphaten, Alkalimetallcitraten oder physiologisch verträglichen Aminen ausgewählt ist.

11. Wirkstoffformulierung mit verzögerter Freisetzung nach Anspruch 9 oder Anspruch 10, wobei die Base eine Hydroxidbase ist, vorzugsweise wobei die Base Natriumhydroxid ist.

12. Wirkstoffformulierung mit verzögerter Freisetzung nach einem der vorhergehenden Ansprüche, wobei die Außenschicht ein Gemisch des magensaftresistenten Polymers und eines enzymatisch abbaubaren Polymers, das von Dickdarmenzymen abgebaut wird, umfasst.

13. Wirkstoffformulierung mit verzögerter Freisetzung nach Anspruch 12, wobei das enzymatisch abbaubare Polymer und das magensaftresistente Polymer im Außenüberzug in einem Verhältnis von mehr als 10:90 vorliegen.

14. Wirkstoffformulierung mit verzögerter Freisetzung nach Anspruch 12 oder Anspruch 13, wobei das magensaftresistente Polymer in der Außenschicht in einer Menge von 3 bis 10 mg/cm², bezogen auf das Trockengewicht des magensaftresistenten Polymers, vorliegt.

15. Wirkstoffformulierung mit verzögerter Freisetzung nach einem der vorhergehenden Ansprüche, wobei die Isolationsschicht ein filmbildendes nichtionisches Polymer, vorzugsweise dasselbe filmbildende nichtionische Polymer, wie es in der Innenschicht vorliegt, umfasst.

16. Verfahren zur Produktion einer Wirkstoffformulierung mit verzögerter Freisetzung zur oralen Verabreichung, um einen Wirkstoff an den Dickdarm abzugeben, nach Anspruch 1, wobei das Verfahren umfasst:
Bilden eines Kerns, der einen Wirkstoff umfasst;
anfängliches Überziehen des Kerns mit einem Isolationsschichtüberzugspräparat, umfassend ein filmbildendes nichtionisches Polymer in einem Lösungsmittel, um einen isolierten Kern zu bilden;
Lösen eines filmbildenden nichtionischen Polymers, das in Darm- oder Gastrointestinalflüssigkeit löslich ist, in einem wässrigen Lösungsmittel mit einem Puffer in einer Menge von mehr als 20 Gew.-% bis 60 Gew.-%, bezogen auf das Trockengewicht des nichtionischen Polymers, um ein Innenschichtüberzugspräparat mit einem pH-Wert von höher als pH 7 zu bilden;
Überziehen des isolierten Kerns unter Verwendung des Innenschichtüberzugspräparats, um einen überzogenen Innenschichtkern zu bilden; und
Überziehen des überzogenen Innenschichtkerns mit einem Außenschichtüberzugspräparat, umfassend ein filmbildendes magensaftresistentes Polymer mit einem pH-Schwellenwert von pH 6 oder höher in einem Lösungsmittelsystem, um einen äußeren überzogenen Kern zu bilden.

17. Verfahren nach Anspruch 16, wobei das Verfahren ein Zugeben einer Base zu dem Innenschichtüberzugspräparat in einer Menge, die dazu ausreicht, den pH-Wert auf das erforderliche Niveau zu erhöhen, umfasst.

18. Verfahren nach Anspruch 17, wobei die Menge der Base, die zu dem Innenschichtüberzugspräparat zugegeben wird, dazu ausreicht, den pH-Wert des Innenschichtüberzugspräparats zu erhöhen, so dass er in einem Bereich von pH 7,5 bis pH 10 liegt.

19. Verfahren nach Anspruch 17 oder Anspruch 18, wobei die Base aus der Gruppe bestehend aus Hydroxidbasen, Alkalimetallbicarbonaten, Alkalimetallcarbonaten, Alkalimetallphosphaten, Alkalimetallcitraten oder physiologisch verträglichen Aminen ausgewählt ist.

20. Verfahren nach einem der Ansprüche 17 bis 19, wobei die Base eine Hydroxidbase ist, vorzugsweise wobei die Base Natriumhydroxid ist.

21. Verfahren nach einem der Ansprüche 16 bis 20, wobei das nichtionische Polymer ein nichtionisches Polymer auf Cellulosebasis ist, vorzugsweise wobei das nichtionische Polymer aus Methylcellulose (MC), Hydroxypropylcellulose (HPC) und Hydroxypropylmethylcellulose (HPMC) ausgewählt ist.

22. Verfahren nach Anspruch 21, wobei das nichtionische Polymer HPMC ist.

23. Verfahren nach einem der Ansprüche 16 bis 22, wobei der Kern mit dem Innenschichtüberzugspräparat überzogen wird, bis das nichtionische Polymer auf den Kern in einer Menge von 2 bis 5 mg/cm², bezogen auf das Trockengewicht des nichtionischen Polymers, überzogen ist.

24. Verfahren nach einem der Ansprüche 16 bis 23, wobei der Puffer in dem Innenschichtüberzugspräparat in einer Menge von 25 Gew.-% bis 60 Gew.-%, bezogen auf das Trockengewicht des nichtionischen Polymers, vorliegt.

25. Verfahren nach einem der Ansprüche 16 bis 24, wobei der Puffer aus der Gruppe bestehend aus einer Carbonsäure mit von 1 bis 16 Kohlenstoffatomen, einem Alkalimetallsalz, einem Erdalkalimetallsalz, einem Ammoniumsalz und einem löslichen Metallsalz ausgewählt ist.

26. Verfahren nach einem der Ansprüche 16 bis 25, wobei der Puffer ein Phosphatsalz ist, vorzugsweise wobei der Puffer Kaliumdihydrogenphosphat ist.

27. Verfahren nach einem der Ansprüche 16 bis 26, wobei das Außenschichtüberzugspräparat ein Gemisch des magensaftresistenten Polymers und eines enzymatisch abbaubaren Polymers, das für einen Angriff durch Dickdarmenzyme anfällig ist, umfasst.

28. Verfahren nach Anspruch 27, wobei das enzymatisch abbaubare Polymer und das magensaftresistente Polymer in dem Außenschichtüberzugspräparat in einem Verhältnis von mehr als 10:90 vorliegen.

29. Verfahren nach Anspruch 27 oder Anspruch 28, wobei der innere überzogene Kern mit dem Außenschichtüberzugspräparat überzogen wird, bis das magensaftresistente Polymer auf den inneren überzogenen Kern in einer Menge von 3 bis 10 mg/cm², bezogen auf das Trockengewicht des magensaftresistenten Polymers, überzogen ist.

30. Verfahren nach einem der Ansprüche 16 bis 29, wobei die Identität des nichtionischen Polymers des Isolationsschichtüberzugspräparats identisch mit der für das nichtionische Polymer des Innenschichtüberzugspräparats ist.

## Revendications

1. Formulation de médicament à libération retardée pour administration orale, destinée à délivrer un médicament au côlon d'un sujet, ladite formulation comprenant :
un noyau, un enrobage du noyau et une couche isolante entre le noyau et l'enrobage,
le noyau comprenant un médicament, l'enrobage comprenant une couche externe et une couche interne,
dans laquelle la couche externe comprend un polymère entérique filmogène ayant un seuil de pH à PH 6 ou au-dessus, et
dans laquelle la couche interne comprend un polymère non ionique filmogène qui est soluble dans le liquide intestinal ou gastro-intestinal, et un agent tampon en une quantité de plus de 20 % en poids à 60 % en poids, par rapport au poids sec du polymère non ionique.

2. Formulation de médicament à libération retardée selon la revendication 1, dans laquelle le polymère non ionique est un polymère non ionique à base de cellulose, de préférence dans laquelle le polymère non ionique est choisi parmi la méthylcellulose (MC), l'hydroxypropylcellulose (HPC) et l'hydroxypropylméthylcellulose (HMPC).

3. Formulation de médicament à libération retardée selon l'une quelconque des revendications précédentes, dans laquelle le polymère non ionique est l'HMPC.

4. Formulation de médicament à libération retardée selon la revendication 1, dans laquelle le polymère non ionique est un polymère acrylate non ionique ou un polymère à base de polyvinyle.

5. Formulation de médicament à libération retardée selon l'une quelconque des revendications précédentes, dans laquelle le polymère non ionique est présent dans la couche interne en une quantité de 2 mg/cm² à 5 mg/cm² sur la base du poids sec du polymère non ionique.

6. Formulation de médicament à libération retardée selon l'une quelconque des revendications précédentes, dans laquelle l'agent tampon est présent dans la couche interne en une quantité de 25 % en poids à 60 % en poids par rapport au poids sec du polymère non ionique dans la couche interne.

7. Formulation de médicament à libération retardée selon l'une quelconque des revendications précédentes, dans laquelle l'agent tampon est choisi dans le groupe constitué par un acide carboxylique ayant de 1 à 16 atomes de carbone, un sel de métal alcalin, un sel de métal alcalino-terreux, un sel d'ammonium et un sel métallique soluble.

8. Formulation de médicament à libération retardée selon l'une quelconque des revendications précédentes, dans laquelle l'agent tampon est un sel phosphate, de préférence dans laquelle l'agent tampon est le dihydrogénophosphate de potassium.

9. Formulation de médicament à libération retardée selon l'une quelconque des revendications précédentes, dans laquelle l'agent tampon est associé à une base.

10. Formulation de médicament à libération retardée selon la revendication 9, dans laquelle la base est choisie dans le groupe constitué par les bases de type hydroxyde, les bicarbonates de métaux alcalins, carbonates de métaux alcalins, phosphates de métaux alcalins, citrates de métaux alcalins, ou les amines physiologiquement tolérées.

11. Formulation de médicament à libération retardée selon la revendication 9 ou la revendication 10, dans laquelle la base est une base de type hydroxyde, de préférence dans laquelle la base est l'hydroxyde de sodium.

12. Formulation de médicament à libération retardée selon l'une quelconque des revendications précédentes, dans laquelle la couche externe comprend un mélange du polymère entérique et d'un polymère dégradable par voie enzymatique qui est dégradé par les enzymes du côlon.

13. Formulation de médicament à libération retardée selon la revendication 12, dans laquelle le polymère dégradable par voie enzymatique et le polymère entérique sont présents dans l'enrobage externe en un rapport de plus de 10:90.

14. Formulation de médicament à libération retardée selon la revendication 12 ou la revendication 13, dans laquelle le polymère entérique est présent dans la couche externe en une quantité de 3 à 10 mg/cm², sur la base du poids sec du polymère entérique.

15. Formulation de médicament à libération retardée selon l'une quelconque des revendications précédentes, dans laquelle la couche isolante comprend un polymère non ionique filmogène, de préférence le même polymère non ionique filmogène que celui présent dans la couche interne.

16. Procédé de production d'une formulation de médicament à libération retardée pour administration orale, destinée à délivrer un médicament au côlon, telle que revendiquée dans la revendication 1, ledit procédé comprenant les étapes consistant à :
former un noyau comprenant le médicament ;
enrober initialement le noyau avec une préparation d'enrobage par couche isolante, comprenant un polymère non ionique filmogène dans un solvant, pour obtenir un noyau isolé ;
dissoudre un polymère non ionique filmogène, qui est soluble dans le liquide intestinal ou gastro-intestinal, dans un solvant aqueux comportant un agent tampon en une quantité de plus de 20 % en poids à 60 % en poids, par rapport au poids sec du polymère non ionique, pour obtenir une préparation d'enrobage par couche interne ayant un pH supérieur à pH 7 ;
enrober le noyau isolé en utilisant la préparation d'enrobage par couche interne, pour obtenir un noyau enrobé de couche interne ; et
enrober le noyau enrobé de couche interne avec une préparation d'enrobage par couche externe comprenant un polymère entérique filmogène ayant un seuil de pH égal à pH 6 ou au-dessus dans un système de solvant, pour obtenir un noyau enrobé de couche externe.

17. Procédé selon la revendication 16, dans lequel le procédé comprend ajouter une base à la préparation de couche d'enrobage interne en une quantité suffisante pour élever le pH jusqu'à la valeur requise.

18. Procédé selon la revendication 17, dans lequel la quantité de base ajoutée à la préparation d'enrobage par couche interne est suffisante pour élever le pH de la préparation d'enrobage par couche interne pour qu'il se situe dans une plage de pH 7,5 à pH 10.

19. Procédé selon la revendication 17 ou la revendication 18, dans lequel la base est choisie dans le groupe constitué par les bases de type hydroxyde, les bicarbonates de métaux alcalins, carbonates de métaux alcalins, phosphates de métaux alcalins, citrates de métaux alcalins, ou les amines physiologiquement tolérées.

20. Procédé selon l'une quelconque des revendications 17 à 19, dans lequel la base est une base de type hydroxyde, de préférence dans lequel la base est l'hydroxyde de sodium.

21. Procédé selon l'une quelconque des revendications 16 à 20, dans lequel le polymère non ionique est un polymère non ionique à base de cellulose, de préférence dans lequel le polymère non ionique est choisi parmi la méthylcellulose (MC), l'hydroxypropylcellulose (HPC) et l'hydroxypropylméthylcellulose (HMPC).

22. Procédé selon la revendication 21, dans lequel le polymère non ionique est l'HMPC.

23. Procédé selon l'une quelconque des revendications 16 à 22, dans lequel le noyau est enrobé avec la préparation d'enrobage par couche interne jusqu'à ce que le polymère non ionique soit appliqué sur le noyau en une quantité de 2 à 5 mg/cm² sur la base du poids sec du polymère non ionique.

24. Procédé selon l'une quelconque des revendications 16 à 23, dans lequel l'agent tampon est présent dans la préparation d'enrobage par couche interne en une quantité de 25 % en poids à 60 % en poids par rapport au poids sec du polymère non ionique.

25. Procédé selon l'une quelconque des revendications 16 à 24, dans lequel l'agent tampon est choisi dans le groupe constitué par un acide carboxylique ayant de 1 à 16 atomes de carbone, un sel de métal alcalin, un sel de métal alcalino-terreux, un sel d'ammonium et un sel métallique soluble.

26. Procédé selon l'une quelconque des revendications 16 à 25, dans lequel l'agent tampon est un sel phosphate, de préférence dans lequel l'agent tampon est le dihydrogénophosphate de potassium.

27. Procédé selon l'une quelconque des revendications 16 à 26, dans lequel la préparation d'enrobage par couche externe comprend un mélange du polymère entérique et d'un polymère dégradable par voie enzymatique qui est susceptible d'être attaqué par les enzymes du côlon.

28. Procédé selon la revendication 27, dans lequel le polymère dégradable par voie enzymatique et le polymère entérique sont présents dans la préparation d'enrobage par couche externe en un rapport de plus de 10:90.

29. Procédé selon la revendication 27 ou la revendication 28, dans lequel le noyau enrobé avec la couche interne est enrobé avec la préparation d'enrobage par couche externe jusqu'à ce que le polymère entérique soit appliqué sur le noyau, enrobé avec la couche interne, en une quantité de 3 à 10 mg/cm², sur la base du poids sec du polymère entérique.

30. Procédé selon l'une quelconque des revendications 16 à 29, dans lequel l'identité du polymère non ionique de la préparation d'enrobage par couche isolante est la même que celle du polymère non ionique de la préparation d'enrobage par couche interne.
